# EUROPEAN PATENT APPLICATION

(11) **EP 1 930 446 A2**
(43) Date of publication of application: **11.06.2008**
(21) Application number: 07121784.8
(22) Date of filing: 28.11.2007
(51) Int. Cl.: C12Q 1/68

(54) **Method for obtaining information regarding quantity of DNA after non-methylated cytosine converting treatment in analysis of DNA methylation**

(30) Priority: 30.11.2006 JP 2006324980
(71) Applicant: SYSMEX CORPORATION, Kobe-shi, Hyogo 651-0073 (JP)
(72) Inventor: Noriaki, Yamamoto, Kobe-shi Hyogo 651-0073 (JP); Masahiro, Kajita, Kobe-shi Hyogo 651-0073 (JP); Takayuki, Takahata, Kobe-shi Hyogo 651-0073 (JP); Ayako, Sakai, Kobe-shi Hyogo 651-0073 (JP); Hideki, Ishihara, Kobe-shi Hyogo 651-0073 (JP)
(74) Representative: De Clercq, Ann G. Y.

(57) **Abstract**

The present invention provides an easy and accurate method for obtaining information regarding a quantity of DNA included in a sample used for analyzing a methylation of a target DNA, comprising steps of: treating a biological sample containing DNA including the target DNA with a non-methylated cytosine converting agent for converting non-methylated cytosine of the DNA into another base; and obtaining information regarding a quantity of DNA included in the sample treated in the treating step by using an oligonucleotide complementary to a cytosine-free region of the target DNA.

## Description

### FIELD OF THE INVENTION

The present invention relates to a method for obtaining information regarding a quantity of DNA after non-methylated cysteine treatment used in analyzing a target DNA as well as a method of calculating the collect rate of the DNA.

### BACKGROUND

In a chromosomal DNA of a higher eukaryote, a 5-position of C (cytosine) of bases constituting DNA undergoes methylation modification in some cases. This methylation of DNA in a higher eukaryote functions as a mechanism for suppressing expression of genetic information. In a genomic DNA, a region containing a large amount of CpG (CpG island, CG island) is present in a promoter region of a gene in many cases. For this reason, when a CpG island has been methylated, a transcription factor can not bind to a promoter, and transcription of a gene is suppressed. When a CpG island has not undergone methylation, it becomes possible for a transcription factor to bind to a promoter region, resulting in the state where a gene can be transcribed. Controlling of gene expression by such methylation of DNA plays an important role in various physiological or pathological phenomena such as early embryo development, tissue-specific gene expression, gene stenciling or inactivation of an X chromosome which is a characteristic phenomenon in a mammal, stabilization of a chromosome, and timing of DNA replication. Further, in recent years, it has been revealed that this DNA methylation is strongly involved in cancer and other diseases.

As a method for analyzing DNA methylation, methylation-specific polymerase chain reaction (methylation-specific PCR) is generally used (see James G. Herman et al., Methylation-specific PCR: A novel PCR assay for methylation status of CpG islands, Proc. Natl. Acad. Sci. USA, Vol. 93, pp. 9821-9826, September 1996). In this method, DNA in a biological sample should be subjected to converting treatment thereby converting non-methylated cytosine into another base. However, it is known that when this converting treatment (that is, treatment of converting cytosine into another base (for example, treatment that involves contacting, with DNAs, a base-converting reagent (non-methylated cytosine converting agent) such as bisulfite)) is conducted, the majority of DNAs in a biological sample are degraded by chemical reaction.

A quantity of DNA in a biological sample can be confirmed by a nucleic-acid amplification method of using oligonucleotides such as a primer and a probe hybridizing with the DNA. When the DNA in the biological sample is subjected to non-methylated cytosine converting treatment, a nucleotide sequence of the DNA in the biological sample is changed during the converting treatment, thus making the sequence after the treatment different from that before the treatment. It follows that before and after the converting treatment, the quantity of the DNA in the biological sample cannot be measured with the same primer or probe. Accordingly, when the collect rate of DNA after non-methylated cytosine converting treatment is determined by PCR, it is necessary that primers used after the treatment are different from those before the treatment, and with the respective primers given, calibration curves are prepared respectively. Use of different primers before and after the treatment leads to problems such as troublesome operation and reduced accuracy of results.

### SUMMARY

The scope of the present invention is defined solely by the appended claims, and is not affected to any degree by the statements within this summary.

The present invention was made in light of these circumstances, and an object of the present invention is to provide a method for easily and accurately obtaining information regarding a quantity of DNA in a biological sample after non-methylated cytosine converting treatment (non-methylated cytosine converted sample), a method for calculating the collect rate of the DNA, and a kit using the same for analysis of DNA methylation.

Another object of the present invention is to provide a method for obtaining information regarding the quantity of DNA in a non-methylated cytosine converted sample which is obtained from a biological sample prepared by a simplified and more rapid pretreatment method for preparing a sample for analysis of DNA methylation, as well as a method for calculating the collect rate of the DNA.

That is, the present invention provides:
(1) A method for obtaining information regarding a quantity of DNA included in a sample used for analyzing a methylation of target DNA, comprising steps of: (a) treating a biological sample containing DNA including the target DNA with a non-methylated cytosine converting agent for converting non-methylated cytosine of the DNA into another base; and (b) obtaining information regarding a quantity of DNA included in the sample treated in the step (a), by using an oligonucleotide complementary to a cytosine-free region of the target DNA;
(2) The method according to (1), wherein the non-methylated cytosine converting agent is bisulfite;
(3) The method according to (1) or (2) further comprising steps of: (c) mixing a solution containing a surfactant and an analyte containing a cell; (d) releasing DNA from the cell into the solution by performing physical treatment; (e) precipitating a insoluble matter by centrifugation; and (f) obtaining a supernatant as the biological sample;
(4) The method according to (3), wherein the cell is tumor cell;
(5) The method according to (3) or (4), wherein the surfactant is sodium cholate or sodium dodecyl sulfate;
(6) The method according to any one of (1) to (5), wherein the oligonucleotide is a primer and/or a probe, and the step (b) is performed so as to obtain the information by DNA amplification using the primer and/or the probe;
(7) The method according to any one of (1) to (6) further comprising a step (g) for judging whether or not a sufficient quantity of the DNA for analyzing methylation of the target DNA is included in the sample on the basis of the information;
(8) The method according to (7), wherein the information is the quantity of the DNA included in the sample treated in the step (a), and the step (g) is preformed so as to judge whether or not the sufficient quantity of the DNA is included in the sample treated in the step (a) by comparing the information and a threshold;
(9) The method according to any one of (1) to (8) further comprising steps of: (h) obtaining second information regarding a quantity of DNA included in a biological sample by using the oligonucleotide; and (i) judging whether or not a sufficient quantity of the DNA for analyzing methylation of the target DNA is included in the biological sample on the basis of the second information;
(10) The method according to (9), wherein the second information is the quantity of the DNA included in the biological sample, and the step (i) is preformed so as to judge whether or not the sufficient quantity of DNA is included in the biological sample or not by comparing the second information and a threshold;
(11) A method for calculating a collect rate of DNA included in a sample used for analyzing a methylation of a target DNA after non-methylated converting treatment, comprising steps of:
   (a) measuring a quantity of DNA included in a biological sample by using an oligonucleotide complementary to a cytosine-free region of the target DNA; (b) treating the biological sample with a non-methylated cytosine converting agent for converting non-methylated cytosine of the DNA into another base; (c) measuring a quantity of DNA included in the sample treated in the step (b) by using the oligonucleotide; and (d) calculating the collect rate of the DNA after the step (b), on the basis of the quantities of the DNA included in the biological sample and in the sample treated in the step (b);
(12) The method according to (11), wherein the non-methylated cytosine converting agent is bisulfite;
(13) The method according to (11) or (12) further comprising steps of: (e) mixing a solution containing a surfactant and an analyte containing a cell; (f) releasing DNA from the cell into the solution by performing physical treatment; (g) precipitating an insoluble matter by centrifugation; and (h) obtaining a supernatant as the biological sample;
(14) The method according to (13), wherein the cell is tumor cell;
(15) The method according to (13) or (14), wherein the surfactant is sodium cholate or sodium dodecyl sulfate;
(16) The method according to any one of (11) to (15), wherein the oligonucleotide is a primer and/or a probe, and the step (a) and the step (c) are performed so as to measure the quantity of the DNA included in the biological sample and in the sample treated in the step (b) by DNA amplification using the primer and/or the probe;
(17) A kit for detecting a methylation of a target DNA comprising:
   a non-methylated cytosine converting agent for converting a non-methylated cytosine of DNA into another base; and an oligonucleotide which is complementary to a cytosine-free region of the target DNA;
(18) The kit according to (17) further comprising a primer for a methylation-specific PCR;
(19) The kit according to (17) or (18), wherein the oligonucleotide is a primer and/or a probe;
(20) The kit according to any one of (17) to (19) further comprises a reagent including a surfactant for preparing the biological sample;

According to the present invention, the same oligonucleotide (primer and/or probe) can be used before and after non-methylated cytosine converting treatment for obtaining information regarding a quantity of DNA included in a sample. Accordingly, the present invention can provide a method for easily and accurately obtaining information regarding the quantity of the DNA included in the sample used in analyzing methylation of a target DNA, a method for calculating the collect rate of DNA, and a kit using these methods for analysis of methylation of DNA.

The method for obtaining the information regarding the quantity of DNA and the method for calculating the collect rate of DNA according to the present invention and the kit for analyzing the methylation of DNA by using these methods are useful for a biological sample in which DNA is not purified, thus making quantification the DNA by absorbance infeasible due to impurities contained therein. The present invention is useful particularly for a biological sample prepared by mixing a biological sample containing a cell with a sample-treating solution containing a surfactant for solubilizing the cell.

### BRIEF DESCRIPTION OF THE DRAWINGS

FIG. 1 shows a result of analysis of a calibration curve of a sample before bisulfite treatment by quantitative real-time PCR with a primer set of SEQ ID NOS: 7 and 8.
FIG. 2 shows a result of analysis of a calibration curve of a sample after bisulfite treatment by quantitative real-time PCR with a primer set of SEQ ID NOS: 7 and 8.
FIG. 3 shows a result of analysis of a calibration curve of a sample before bisulfite treatment by quantitative real-time PCR with a primer set of SEQ ID NOS: 7 and 8 and a fluorescence-labeled probe method with the probe of SEQ ID NO: 11.
FIG. 4 shows a result of analysis of a calibration curve of a sample after bisulfite treatment by quantitative real-time PCR with a primer set of SEQ ID NOS: 7 and 8 and a fluorescence-labeled probe method with the probe of SEQ ID NO: 11.

### DETAILED DESCRIPTION OF THE EMBODIMENT

The biological sample in this embodiment is not particularly limited insofar as it contains DNA. It is preferably a biological sample containing a genomic DNA in the living body. Particularly, it is preferably a biological sample prepared from a clinical sample. The clinical sample includes blood, plasma, lymph fluid, urine, nipple secretory fluid, and tissues collected by surgery and biopsy. It is particularly preferably a tumor cell-containing clinical sample.

Because a genomic DNA occurs in a cell, the genomic DNA is preferably released from the cell into a solution. For example, a genomic DNA can be extracted and purified with a commercial kit. Alternatively, an intracellular DNA can be released into a solution by mixing a sample-treating solution containing a cell-solubilizing surfactant with a cell-containing sample followed by physical treatment thereof (for example, stirring, homogenization, sonication etc.). After physical treatment, broken cells are precipitated by centrifugation, and the genomic DNA-containing supernatant thus obtained can be used as a biological sample. By using the sample-treating solution, the extraction, purification etc. of genomic DNA with the above kit becomes unnecessary. Accordingly, a biological sample can be obtained rapidly and easily through mere centrifugation only. The surfactant used herein is not particularly limited insofar as it can solubilize cells. For example, sodium cholate, sodium dodecyl sulfate (SDS) etc. can be preferably used as the surfactant.

In this embodiment, the non-methylated cytosine converting agent refers to a substance that converts non-methylated cytosine included in DNA into a base other than cytosine. A bisulfite is preferably used as the non-methylated cytosine converting agent. As the bisulfite, it is possible touse sodium bisulfite, potassium bisulfite, calcium bisulfite, magnesium bisulfite or the like. When DNA is treated with a bisulfite, non-methylated cytosine in the DNA is converted via deamination into uracil. The non-methylated cytosine converting agent does not act on methylated cytosine in the DNA and does not cause conversion of the base. Preferably, the non-methylated cytosine converting agent is dissolved in a solvent and used as a solution.

When a bisulfite is used as the non-methylated cytosine converting agent, the amount of the bisulfite added is not particularly limited within such a range as to sufficiently convert non-methylated cytosine of DNA in a biological sample. None-methylated cytosine converting may be carried out for a period of time at a temperature depending on the concentration of the bisulfite, in such a range that non-methylated cytosine can be sufficiently converted. For example, the concentration of the bisulfite in non-methylated cysteine converting treatment is 1 M or more, preferably 1 to 15 M, more preferably 3 to 10 M. Specifically, when 6 M sodium bisulfite is added to a biological sample, non-methylated cytosine can be converted into uracil by incubation for 10 to 90 minutes at 50 to 80°C. The non-methylated cytosine converting agent is not limited to the bisulfite. Other non-methylated cytosine converting agents that convert non-methylated cytosine specifically into another base can also be used.

In this embodiment, the non-methylated cytosine converted sample refers to a sample obtained by treating DNA in a biological sample with a non-methylated cytosine converting agent. For example, a sample obtained by treating a genomic DNA in a biological sample with a bisulfite can be mentioned.

In this embodiment, a cytosine-free region in a nucleotide sequence of DNA as a subject of methylation analysis (a target DNA) is not particularly limited insofar as it is a region which does not contain cytosine in the nucleotide sequence. The cytosine-free region in the nucleotide sequence of the target DNA is preferably as follows:
i) a region having a nucleotide sequence of 15 or more bases, particularly 17 or more bases;
ii) a region having a nucleotide sequence free from preferably 3 or more consecutive guanine bases, particularly 4 or more consecutive guanine bases; and
iii) a region having a nucleotide sequence free from unknown bases.

For example, cytosine-free regions in a nucleotide sequence on human chromosome 14 include the following nucleotide sequences:
SEQ ID NO 1: TAGAATAGGGAGTAGAGAAAAAGTAGGAAGATGAGTTAGAAGG
SEQ ID NO 2: ATTGGGATGTGTAAGAGATGAAGGAGAGTTTAGA
SEQ ID NO 3: AGTGGATTGGATGTGATGTTGGAGAAA
SEQ ID NO 4: AAAGAGATTGGAGGTAAGAGGGAGA
SEQ ID NO 5: TAGATTTTGGATGTATGTTTATGTTGAGATGTTGGAG
SEQ ID NO 6: TGAGATGGAGAAAAGTGATGTTTAGAAGG

For nucleotide sequences of human chromosomal DNAs, databases in research institutes such as National Center for Biotechnology Information (NCBI), University of California at Santa Cruz (UCSC), etc. can be referred to.

In this embodiment, the oligonucleotide complementary to a cytosine-free region of a target DNA refers to an oligonucleotide substantially or fully complementary to a cytosine-free region in a nucleotide sequence of DNA as a subject of methylation analysis. This oligonucleotide can be used as a primer or probe.

The term "substantially complementary" indicates that the mentioned oligonucleotide is 80% or more, preferably 90% or more and particularly preferably 95% or more complementary to the nucleotide sequence of DNA as a subject of methylation analysis.

In this embodiment, a method for measuring the quantity of DNA included in a non-methylated cytosine converted sample by using an oligonucleotide complementary to a cytosine-free region of a target DNA is not particularly limited insofar as it is a method wherein the quantity of DNA included in a sample can be measured by using the above-mentioned oligonucleotide as a primer or probe. For example, the following methods i) and ii) can be mentioned as known methods.
i) A method for measuring the quantity of DNA included in a sample, from a result of amplification of the DNA in the sample with the oligonucleotide as a primer.
ii) A method for measuring the quantity of DNA included in a sample by hybridizing the oligonucleotide as a labeled probe, with the DNA in the sample, and then measuring the intensity of a signal from the labeled probe hybridized with the DNA.

Particularly, the method for measuring the quantity of DNA in a sample from the amplification result of the DNA is preferable.

Hybridization used herein is not particularly limited insofar as after hybridization of DNA in a sample with a labeled probe, the intensity of a signal from the labeledprobe hybridized with the DNA can be measured. Examples of such hybridization include Southern hybridization, array hybridization, affinity chromatography, and *in situ* hybridization. Particularly, Southern hybridization and array hybridization are preferable.

A label for a labeled probe used in hybridization may be a known label. Examples of such label include radioisotopes, fluorescent substances, chemical substances, antigens, antibodies and enzymes. More specific examples include radioisotopes such as ³²P, fluorescent substances such as FITC (fluorescence isothiocyanate) and rhodamine, haptens such as digoxigenin, enzymes such as alkali phosphatase and peroxidase, and biochemical substances such as biotin. These labels can be introduced into probes by known methods. Reagents etc. for introducing labels into probes are easily commercially available.

In this embodiment, the method for amplifying DNA is not particularly limited insofar as it is a known method for amplifying DNA by using primers. The method includes, for example, PCR, LAMP, ICAN, SDA and TAS. Particularly, PCR is preferable.

In this embodiment, the primer complementary to a cytosine-free region of a target DNA is substantially or fully complementary to the cytosine-free region in a nucleotide sequence of DNA as a subj ect of methylation analysis. The primer can initiate an extension reaction of the DNA in DNA amplification step. For example, preferable primers for amplification of DNA by PCR are those satisfying i) to iv):
i) the percentage of guanine bases in the primer nucleotide sequence is preferably 30% to 70%, particularly preferably 40% to 60%,
ii) the primer is preferably a 15- to 35-mer, particularly preferably 17 bp to 30 bp in length,
iii) the difference in Tm value between primers at both ends is preferably within 5°C, particularly preferably within 2°C,
iv) preferably a 3'-terminal 3-mer, particularly preferably 5-mer, of the primer is fully complementary to a region with which the primer hybridizes.

Specific nucleotide sequences of the primers include nucleotide sequences of SEQ ID NOS: 7 and 8 below.
SEQ ID NO 7: AGGGAGTAGAGAAAAAGTAGGAAGATGAGT
SEQ ID NO 8: TCCAACATCACATCCAATCCA

The primers represented by SEQ ID NOS: 7 and 8 amplify the 143-bp region of human chromosome 14 which is represented by SEQ ID NO: 9 below.

SEQ ID NO 9:

AGGGAGTAGAGAAAAAGTAGGAAGATGAGTTAGAAGGCTATCATTGGGATGTGTAAGAG

ATGAAGGAGAGTTTAGACCAGGGTGATAATGAGTGTGTTGGGAAATAGACGAAGATAGA

ACAGTGGATTGGATGTGATGTTGGA

As is evident from SEQ ID NO: 9,
AGGGAGTAGAGAAAAAGTAGGAAGATGAGT and TGGATTGGATGTGATGTTGGA that are regions with which the primers of SEQ ID NOS: 7 and 8 hybridize, do not contain cytosine. Accordingly, even if non-methylated cytosine is converted into another base by the non-methylated cytosine converting agent, the regions with which the primers hybridize are not influenced. Hence, PCR can be accomplished where the same primers are used for DNA before and after treatment with the non-methylated cytosine converting agent. Therefore, the procedure for measuring the quantity of DNA is made easy. Operation mistakes such as use of wrong primers can be prevented. Accurate measurement of DNA is made feasible without the change in DNA amplification efficiency which may be caused by use of different primers.

The full length of DNA amplified by PCR is preferably 60 bp to 400 bp, particularly preferably 60 bp to 200 bp, from the viewpoint of the efficiency of PCR.

The PCR method in this embodiment is not particularly limited insofar as primers complementary respectively to cytosine-free regions of a target DNA can be used to amplify the DNA. Quantitative PCR is preferable. Particularly, quantitative real-time PCR is preferable from the viewpoint of accurate measurement of the quantity of DNA in a biological sample.

As the method for measuring the quantity of DNA from the amplification result of the DNA by PCR, a known method can be used. Examples include:
i) a method wherein a control and a biological sample are subjected under the same conditions to PCR, then to agarose electrophoresis, and compared with each other with respect to the signal intensity of a band of each amplified DNA, thereby measuring the quantity of the DNA (agarose gel electrophoresis);
ii) a method wherein DNA-binding dye such as SYBR Green which emits fluorescence upon binding to a double-stranded DNA is used to measure an increase in fluorescence by PCR, thereby measuring the quantity of the DNA (SYBR Green method); and
iii) a method wherein in the course of synthesizing DNA by PCR with a fluorescence-labeled RNA probe binding complementarily to a certain area within an amplification region, fluorescence emitted by the fluorescence-labeled RNA probe upon degradation is measured thereby measuring the quantity of the DNA (fluorescence-labeled probe method). Reagents, apparatus etc. used in these methods are commercially available.

When the fluorescence-labeled probe method is used in measuring the quantity of DNA from the amplification result of the DNA by PCR, a fluorescence-labeled probe complementary to a cytosine-free region in a nucleotide sequence of the DNA as a subject of methylation analysis is preferably used.

In this case, the fluorescence-labeled probe is preferably a 15- to 50-mer. It is particularly preferably a 20- to 40-mer. The difference in Tm between the fluorescence-labeled probe and the primer is preferably 5 to 10°C. The difference is particularly preferably 8 to 10°C.

A specific nucleotide sequence of the fluorescence-labeled probe includes SEQ ID NO: 10 below. SEQ ID NO 10: ATTGGGATGTGTAAGAGATGAAGGAGAGTTTAGA

The nucleotide sequence of SEQ ID NO: 10 for the florescence-labeled probe can hybridize with the 143-bp region on human chromosome 14 which is represented by SEQ ID NO: 9 amplified with the primers of SEQ ID NOS: 7 and 8.

SEQ ID NO 9:

AGGGAGTAGAGAAAAAGTAGGAAGATGAGTTAGAAGGCTATCATTGGGATGTGTAAGAG

ATGAAGGAGAGTTTAGACCAGGGTGATAATGAGTGTGTTGGGAAATAGACGAAGATAGA

ACAGTGGATTGGATGTGATGTTGGA

As is evident from SEQ ID NO: 9,
ATTGGGATGTGTAAGAGATGAAGGAGAGTTTAGA which is a region to be hybridized with the fluorescence-labeled probe represented by SEQ ID NO: 10 does not contain cytosine. Accordingly, the region to be hybridized with the fluorescence-labeled probe is not influenced even if non-methylated cytosine is converted into another base by the non-methylated cytosine converting agent.

It follows that before and after treatment of DNA with the non-methylated cytosine converting agent, the quantity of the DNA can be measured by the fluorescence-labeled probe method using the same fluorescence-labeled probe.

In design of primers and measurement of the quantity of the DNA in LAMP, ICAN, SDA or TAS other than PCR, the primers optimum for amplification of each DNA and the optimum DNA measurement method may be suitably selected from known methods, in consideration of the PCR techniques described above.

For example, when LAMP is used, primers can be designed with LAMP primer design-support software Primer Explorer (Eiken Chemical Co., Ltd.). Specifically, objective primers can be designed on condition that the primers are complementary respectively to cytosine-free regions in a nucleotide sequence of DNA as a subject of methylation analysis. Then, the quantity of the DNA can be measured easily with a real-time turbidity measuring instrument LA-200 (Eiken Chemical Co., Ltd.) etc.

The information regarding a quantity of DNA in this embodiment is not particularly limited insofar as the information can represent the quantity of the DNA directly or indirectly. In other wards, the information includes not only a measurement result of the quantity of the DNA but also a signal intensity of a band, turbidity and a fluorescence intensity which are used for measuring the quantity of the DNA.

Measurement of DNA after non-methylated cytosine converting treatment in this embodiment is not particularly limited insofar as the quantity of the whole DNA in a non-methylated cytosine converted sample can be determined from the measurement result of the quantity of the DNA included in a part of the non-methylated cytosine converted sample. For example, when the quantity of a non-methylated cytosine-converted sample used to measure the quantity of DNA therein is 1/10 relative to the whole sample, the measurement result of the quantity of the DNA in the non-methylated cytosine-converted sample can be multiplied by 10 to determine the quantity of the DNA included in the whole sample.

When the quantity of DNA included in a non-methylated cytosine converted sample is smaller than a predetermined amount, this sample can be judged not to have a sufficient quantity of DNA to detect DNA methylation. On the other hand, when the quantity of DNA included in a non-methylated cytosine converted sample is equal to, or larger than, a predetermined amount, this sample can be judged to have a sufficient quantity of DNA to detect DNA methylation. The predetermined amount is preferably an empirically established threshold. For example, when the quantity of DNA included in a non-methylated cytosine converted sample is less than 10 copies, the sample can be judged not to have a sufficient quantity of DNA to detect DNA methylation.

When DNA included in a non-methylated cytosine converted sample is amplified by using the above mentioned primers, it is also possible to judge whether or not a sufficient quantity of DNA for analyzing methylation of a target DNA is included in the non-methylated cytosine converted sample based on a result of DNA amplification. If the DNA included in the non-methylated cytosine converted sample is amplified by the primers, the sample can be judged to have a sufficient quantity of DNA for analyzing methylation of the target DNA. On the contrary, if the DNA is not amplified by the primers, the sample can be judged not to have a sufficient quantity of DNA. The amplified DNA can be confirmed by a known method. For example, it is easily confirmed by performing agarose gel electrophoresis to a part of a non-methylated cytosine converted sample. In this way, it is possible to judge whether the sufficient quantity of the DNA for analyzing methylation of the target DNA is included in the non-methylated cytosine converted sample or not without measuring the quantity of the DNA included in the non-methylated cytosine converted sample.

The reliability of information on detection of DNA methylation can also be judged on the basis of the judgment result mentioned above. For example, information on detection of DNA methylation obtained from a sample judged not to have a sufficient quantity of DNA to detect DNA methylation can be judged to be poor in reliability.

Preferably, the quantity of DNA included in a living sample is also measured. The same oligonucleotide as used in measuring the quantity of DNA included in the above-mentioned non-methylated cytosine converted sample can be used to measure the quantity of DNA included in a biological sample. On the basis of the quantity of DNA included in the biological sample, it is also possible to judge whether or not a sufficient quantity of DNA to detect DNA methylation is included in the biological sample. For example, when the quantity of DNA included in a non-methylated cytosine converted sample is less than 10 copies, the sample can be judged not to have a sufficient quantity of DNA to detect DNA methylation.

In the same way of the non-methylated cytosine converted sample, it is possible to judge whether or not a sufficient quantity of DNA for analyzing methylation of a target DNA is included in the biological sample based on a result of DNA amplification using the primers. For example, a biological sample can be judged to have a sufficient quantity of DNA for analyzing methylation of the target DNA when the DNA is amplified.

The same oligonucleotides (primers and/or a probe) can be used to judge whether or not a non-methylated cytosine converted sample and a biological sample contain a sufficient quantity of DNA to detect methylation of a target DNA. Accordingly, the judgment becomes easy and accurate.

The collect rate of DNA after non-methylated cytosine converting treatment in this embodiment can be confirmed on the basis of the quantity of DNA included in a biological sample and in a non-methylated cytosine converted sample. Specifically, the collect rate of DNA can be determined from a first measurement result which is a measurement result of the quantity of DNA included in a biological sample measured before treatment with a non-methylated cytosine converting agent and a second measurement result which is a measurement result of the quantity of DNA included in a non-methylated cytosine converted sample after treatment with a non-methylated cytosine converting agent.

The collect rate of DNA in a biological sample in this embodiment is not particularly limited insofar as the ratio of the quantity of DNA in a non-methylated cytosine converted sample after addition of a non-methylated cytosine converting agent, to the quantity of DNA in a biological sample before addition of a non-methylated cytosine converting agent can be shown. For example, when the quantity of DNA in a biological sample is 200 ng and the quantity of DNA in a non-methylated cytosine converted sample is 100 ng, the collect rate of the DNA is 50%.

In this embodiment, the primers for methylation-specific PCR are specific primers used for analysis of DNA methylation or non-methylation by treatment with a non-methylated cytosine converting agent for converting non-methylated cytosine into another base. The primers for methylation-specific PCR are known and also commercially available.

A kit for analysis of DNA methylation, comprising primers for methylation-specific PCR, is also known. The kit for analysis of DNA methylation in this embodiment can also be constituted by adding, to the constitution of the above kit for analysis of DNA methylation, the oligonucleotide complementary to a cytosine-free region in a nucleotide sequence of DNA as a subject of methylation analysis in this embodiment.

### EXAMPLES

### Example 1

### <Preparation of primers and a fluorescence-labeled probe used in quantitative real-time PCR>

### 1) Selection of genome sequences not undergoing the conversion of DNA sequence by treatment with a bisulfite solution

DNA sequences free of a base (cytosine, C) converted by bisulfite treatment were selected from among about 3×10 ^ [sic] 9 bp genome sequences by analysis with a computer using a program prepared for identifying sequences satisfying the following conditions.

Human genome DNA sequences were obtained from a database of NCBI.
Condition 1: C and unknown bases are not included.
Condition 2: The number of bases is 17 or more.
Condition 3: 4 or more consecutive G bases are not included.

### 2) Design of primers for quantitative real-time PCR

From genome sequences selected in the above analysis, nucleotide sequences satisfying the following conditions were selected to determine primer sequences optimum for quantitative real-time PCR.
Condition 4: The full length of a nucleotide sequence of an amplified DNA, including primers at both ends, is in the range of 60 to 150 bp.
Condition 5: The difference in Tm between the primers at both ends is within 2°.
Condition 6: The Tm value of the fluorescence-labeled probe is higher by 8° [inclusive] to 10° [exclusive] than the Tm value of the primer, and any Tm values are 60°C or more.
Condition 7: The proportion of G bases is 40 to 60%.
Condition 8: The primer is a 17- to 30-mer.
Condition 9: The fluorescence-labeled probe is a 20-to 40-mer.

An example of analysis of sequences which on chromosome 14, satisfy the above conditions is shown below. As DNA sequences which on chromosome 14, satisfy Conditions 1 to 3, there were 534888 sequences having a sequence of 17 to 533 bases in length.

From these 534888 sequences, 56846 sequences are selected as candidates for primer or fluorescence-labeled probe sequences by eliminating those sequences wherein:
the proportion of G basses is 30 to 70%,
6 or more sequences each consisting of 1 base (3 types) are continued,
3 or more sequences each consisting of 2 bases (6 types) are continued,
2 or more sequences each consisting of 3 bases (24 types) are continued, and
2 or more sequences each consisting of 4 base (78 types) are continued.

Further, a group of DNA sequences having primer sequences at both ends and having one or more fluorescence-labeled probe sequences between the primer sequences were searched for DNA sequences whose length excluding the primer sequences is 26 (= 60 - 34) to 116 (= 150 - 34) bases. As a result, 568 sets of DNA sequences were selected.

In the selected 568 sets, there were 261 sets satisfying Condition 9 (the probe sequence is 20 bases or more). These DNA sequences were further limited to those having a primer sequence of 25 bases or more and a fluorescence-labeled probe sequence of 30 bases or more, thereby narrowing them down to 2 sets of candidates (first candidate, second candidate) shown in Table 1.

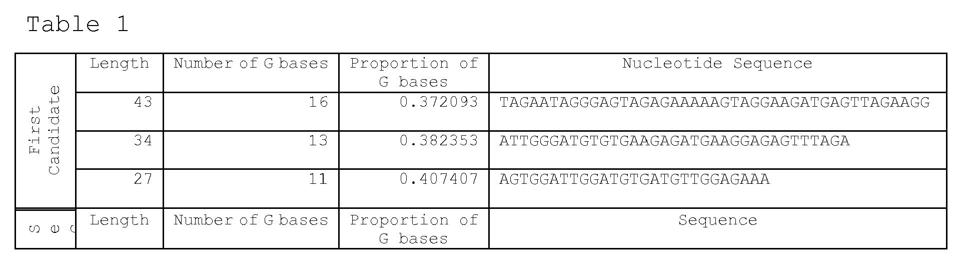

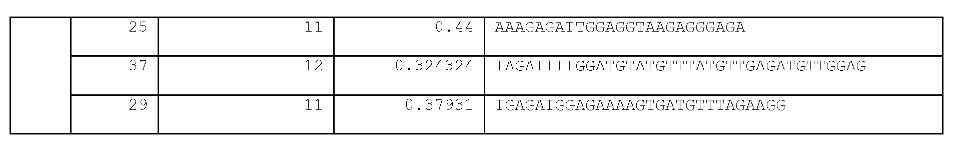

For each of the 2 sets of candidates, the Tm value of the region satisfying Conditions 5 to 8 above was calculated, and those sequences satisfying the conditions were selected as primers.

A set of primers represented by SEQ ID NOS : 7 and 8 amplifying DNA sequence which is a sequence occurring in the human genome sequence, is free of C (cytosine) and is represented by SEQ ID NO: 9, to generate a 143-bp amplification product, were selected. For measuring the quantity of DNA by the fluorescence-labeled probe method, a fluorescence-labeled probe represented by SEQ ID NO: 1 was selected.
<Primer set>
SEQ ID NO 7: AGGGAGTAGAGAAAAAGTAGGAAGATGAGT
SEQ ID NO 8: TCCAACATCACATCCAATCCA
<DNA sequence to be amplified>
SEQ ID NO 9:
AGGGAGTAGAGAAAAAGTAGGAAGATGAGTTAGAAGGCTATCATTGGGATGTGTAAGAG ATGAAGGAGAGTTTAGACCAGGGTGATAATGAGTGTGTTGGGAAATAGACGAAGATAGA ACAGTGGATTGGATGTGATGTTGGA
<Fluorescence-labeled probe>
SEQ ID NO 11: FAM-ATTGGGATGTGTAAGAGATGAAGGAGAGTTTAGA-TAMRA wherein FAM (5-carboxy-fluorescein) is a fluorescent dye, and TAMRA (6-carboxy-tetramethyl rhodamine) is a quencher (excitation energy-absorbing agent).

### Example 2

### <Measurement of the quantity of the DNA by quantitative real-time PCR using a primer set of SEQ ID NOS: 7 and 8>

400 µl of 0.3 M NaOH was added to a genome (2 µg) derived from breast cancer cell strain MDA 231 cells and incubated at 37°C for 10 minutes. Then, 400 µL of 10M sodium bisulfite solution was added to each tube and incubated at 70°C for 40 minutes (converting treatment). DNA included in the solution treated with sodium bisulfite was purified with DNA purification kit (QIAquik^{®} manufactured by Qiagen) to give a biological sample.

Using a primer set of SEQ ID NOS: 7 and 8, the samples before and after bisulfite treatment were subjected to quantitative real-time PCR (MX3500P, Stratagene). As the reagent for quantitative real-time PCR, qPCR reagent (Roche) was used. Conditions for quantitative real-time PCR are shown below.

### <PCR solution>

| | |
|---|---|
| 2xMaster Mix (Roche) | 12.5 µL |
| Primer of SEQ ID NO: 7 (10 µM) | 1 µL |
| Primer of SEQ ID NO: 8 (10 µM) | 1 µL |
| Template DNA | 1 µL |
| Distilled water | 9.5 µL |
| Total | 25 µL |

### <PCR conditions>

| | |
|---|---|
| 95°C | 10 minutes |
| 1 cycle | |
| 95°C | 30 seconds |
| 60°C | 15 seconds |
| 72°C | 30 seconds |
| 40 cycle | |
| 95°C | 30 seconds |
| 30°C | 30 seconds |
| 95°C | 1 minutes |
| 1 cycle | |

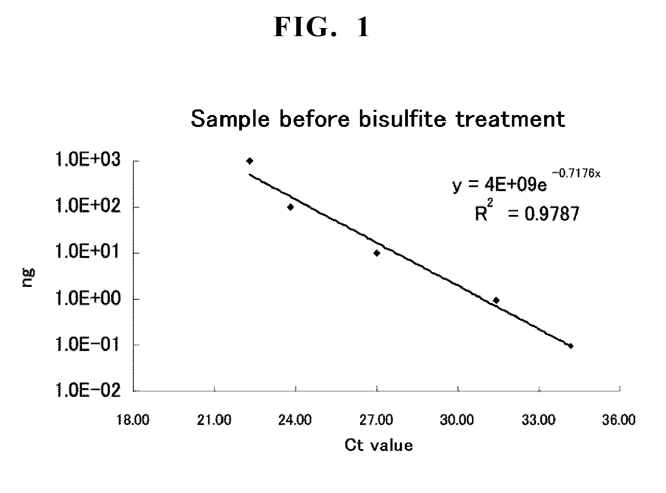

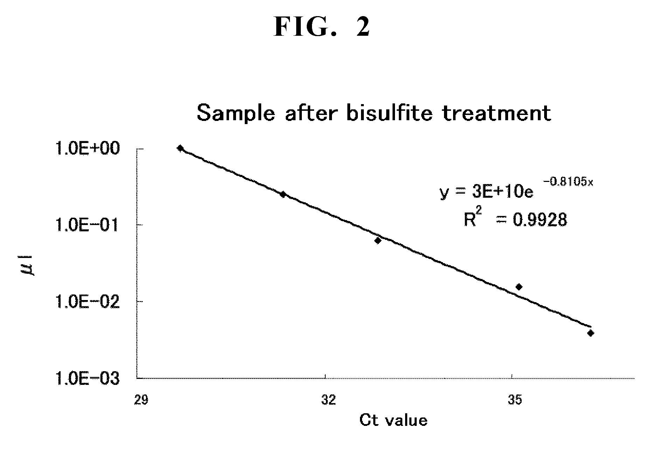

The result of a calibration curve, by quantitative real-time PCR, of the sample before bisulfite treatment is shown in FIG. 1. The result of a calibration curve, by quantitative real-time PCR, of the sample after bisulfite treatment is shown in FIG. 2. As is evident from FIGS. 1 and 2, quantitative real-time PCR with the same primer set (SEQ ID NOS: 7 and 8) is feasible before and after bisulfite treatment. It was thereby proven that the quantity of DNA can be measured without changing the primer set before and after bisulfite treatment.

### Example 3

### <Measurement of the quantity of DNA by quantitative real-time PCR using a primer set of SEQ ID NOS: 7 and 8 and a fluorescence-labeled probe of SEQ ID NO: 11>

Using a primer set of SEQ ID NOS: 7 and 8 and a fluorescence-labeled probe of SEQ ID NO: 11, samples before and after bisulfite treatment prepared in the same manner as in Example 2 were subjected to quantitative real-time PCR (MX3500P, Stratagene). As the reagent for quantitative real-time PCR, qPCR reagent (Roche) was used. Conditions for quantitative real-time PCR are shown below.

### <PCR solution>

| | |
|---|---|
| 2xMaster Mix (Roche) | 12.5 µL |
| Primer of SEQ ID NO: 7 (10 µM) | 1 µL |
| Primer of SEQ ID NO: 8 (10 µM) | 1 µL |
| Probe of SEQ ID NO: 11 (10 µM) | 0.5µL |
| Template DNA | 1 µL |
| Distilled water | 9.5 µL |
| Total | 25 µL |

### <PCR conditions>

| | |
|---|---|
| 95°C | 10 minutes |
| 1 cycle | |
| 95°C | 30 seconds |
| 60°C | 15 seconds |
| 72°C | 30 seconds |
| 40 cycle | |

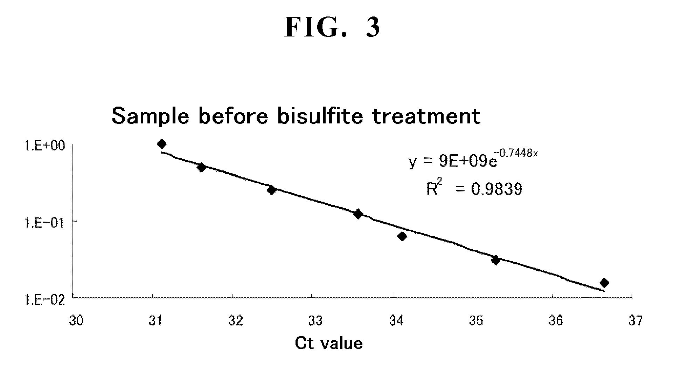

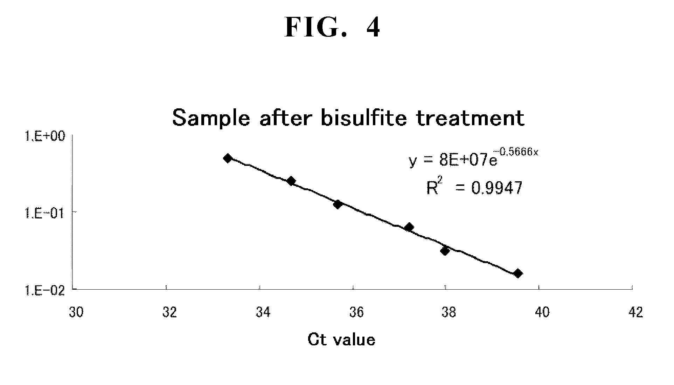

The result of a calibration curve, by quantitative real-time PCR, of the sample before bisulfite treatment is shown in FIG. 3. The result of a calibration curve, by quantitative real-time PCR, of the sample after bisulfite treatment is shown in FIG. 4. It was proven that even when the fluorescence-labeled probe was used, the quantity of the DNA can be measured with the same primer set (SEQ IDNOS: 7 and 8) before and after bisulfite treatment in the same manner as in Example 1.

### Example 4

### <Measurement of the quantity of DNA by quantitative real-time PCR using a primer set of SEQ ID NOS: 7 and 8 and a fluorescence-labeled probe of SEQ ID NO: 12>

Using a fluorescence-labeled probe set forth in SEQ ID NO: 12 below, quantitative real-time PCR using the fluorescence-labeled probe method was conducted in the same manner as in Example 3.

### <Fluorescence-labeled probe>

SEQ ID NO 12: FAM-AGGGTGATAATGAGTGTGTTGGGAAATAGA-TAMRA wherein FAM (5-carboxy-fluorescein) is a fluorescent dye, and TAMRA (6-carboxy-tetramethyl rhodamine) is a quencher (excitation energy-absorbing agent).

Although not shown in the figures, a calibration curve of the sample after and before bisulfite treatment could be prepared by using quantitative real-time PCR even in the case where the fluorescence-labeled probe of SEQ ID NO: 12 was used, similar to the case where the fluorescence-labeled probe of SEQ ID NO: 11 was used. It was thereby revealed that when a fluorescence-labeled probe complementary to a cytosine-free region is used in quantitative real-time PCR using the fluorescence-labeled probe method, the quantity of DNA can be measured with the same primer set.

The foregoing detailed description and examples have been provided by way of explanation and illustration, and are not intended to limit the scope of the appended claims. Many variations in the presently preferred embodiments will be obvious to one of ordinary skill in the art, and remain within the scope of the appended claims and their equivalents.

## Claims

1. A method for obtaining information regarding a quantity of DNA included in a sample used for analyzing a methylation of target DNA, comprising steps of:
(a) treating a biological sample containing DNA including the target DNA with a non-methylated cytosine converting agent for converting non-methylated cytosine of the DNA into another base; and
(b) obtaining information regarding a quantity of DNA included in the sample treated in the step (a), by using an oligonucleotide complementary to a cytosine-free region of the target DNA.

2. The method according to claim 1, wherein the non-methylated cytosine converting agent is bisulfite.

3. The method according to claim 1 or 2 further comprising steps of:
(c) mixing a solution containing a surfactant and an analyte containing a cell;
(d) releasing DNA from the cell into the solution by performing physical treatment;
(e) precipitating a insoluble matter by centrifugation; and
(f) obtaining a supernatant as the biological sample.

4. The method according to claim 3, wherein the cell is tumor cell.

5. The method according to claim 3 or 4, wherein the surfactant is sodium cholate or sodium dodecyl sulfate.

6. The method according to any one of claims 1 to 5, wherein the oligonucleotide is a primer and/or a probe, and the step (b) is performed so as to obtain the information by DNA amplification using the primer and/or the probe.

7. The method according to any one of claims 1 to 6 further comprising a step (g) for judging whether or not a sufficient quantity of the DNA for analyzing methylation of the target DNA is included in the sample on the basis of the information.

8. The method according to claim 7, wherein the information is the quantity of the DNA included in the sample treated in the step (a), and the step (g) is preformed so as to judge whether or not the sufficient quantity of the DNA is included in the sample treated in the step (a) by comparing the information and a threshold.

9. The method according to any of claims 1 to 8 further comprising steps of:
(h) obtaining second information regarding a quantity of DNA included in a biological sample by using the oligonucleotide; and
(i) judging whether or not a sufficient quantity of the DNA for analyzing methylation of the target DNA is included in the biological sample on the basis of the second information.

10. The method according to claim 9, wherein the second information is the quantity of the DNA included in the biological sample, and the step (i) is preformed so as to judge whether or not the sufficient quantity of DNA is included in the biological sample or not by comparing the second information and a threshold.

11. A method for calculating a collect rate of DNA included in a sample used for analyzing a methylation of a target DNA after non-methylated converting treatment, comprising steps of :
(a) measuring a quantity of DNA included in a biological sample by using an oligonucleotide complementary to a cytosine-free region of the target DNA;
(b) treating the biological sample with a non-methylated cytosine converting agent for converting non-methylated cytosine of the DNA into another base;
(c) measuring a quantity of DNA included in the sample treated in the step (b) by using the oligonucleotide; and
(d) calculating the collect rate of the DNA after the step (b), on the basis of the quantities of the DNA included in the biological sample and in the sample treated in the step (b).

12. The method according to claim 11, wherein the non-methylated cytosine converting agent is bisulfite.

13. The method according to claim 11 or 12 further comprising steps of:
(e) mixing a solution containing a surfactant and an analyte containing a cell;
(f) releasing DNA from the cell into the solution by performing physical treatment;
(g) precipitating a insoluble matter by centrifugation; and
(h) obtaining a supernatant as the biological sample.

14. The method according to claim 13, wherein the cell is tumor cell.

15. The method according to claim 13 or 14, wherein the surfactant is sodium cholate or sodium dodecyl sulfate.

16. The method according to any one of claims 11 to 15, wherein the oligonucleotide is a primer and/or a probe, and the step (a) and the step (c) are performed so as to measure the quantity of the DNA included in the biological sample and in the sample treated in the step (b) by DNA amplification using the primer and/or the probe.

17. A kit for detecting a methylation of a target DNA comprising:
a non-methylated cytosine converting agent for converting a non-methylated cytosine of DNA into another base; and
an oligonucleotide which is complementary to a cytosine-free region of the target DNA.

18. The kit according to claim 17 further comprising a primer for a methylation-specific PCR.

19. The kit according to claim 17 or 18, wherein the oligonucleotide is a primer and/or a probe.

20. The kit according to any one of claims 17 to 19 further comprises a reagent including a surfactant for preparing the biological sample.


SEQUENCE LISTING

<110>  SYSMEX CORPORATION

<120>  METHOD FOR OBTAINING INFORMATION REGARDING QUANTITY OF DNA AFTER NON-METHYLATED
CYTOSINE CONVERTING TREATMENT IN ANALYSIS OF DNA METHYLATION

<130>  SYSM-020-EP

<150>  JP 2006-324980
<151>  2006-11-30

<160>  12

<170>  PatentIn version 3.1

<210>  1
<211>  43
<212>  DNA
<213>  Homo sapiens

<400>  1
tagaataggg agtagagaaa aagtaggaag atgagttaga agg                    43


<210>  2
<211>  34
<212>  DNA
<213>  Homo sapiens

<400>  2
attgggatgt gtaagagatg aaggagagtt taga                              34


<210>  3
<211>  27
<212>  DNA
<213>  Homo sapiens

<400>  3
agtggattgg atgtgatgtt ggagaaa                                      27


<210>  4
<211>  25
<212>  DNA
<213>  Homo sapiens

<400>  4
aaagagattg gaggtaagag ggaga                                        25


<210>  5
<211>  37
<212>  DNA
<213>  Homo sapiens

<400>  5
tagattttgg atgtatgttt atgttgagat gttggag                           37


<210>  6
<211>  29
<212>  DNA
<213>  Homo sapiens

<400>  6
tgagatggag aaaagtgatg tttagaagg                                    29


<210>  7
<211>  30
<212>  DNA
<213>  Artificial Sequence

<220>
<223>  Primer

<400>  7
agggagtaga gaaaaagtag gaagatgagt                                        30


<210>  8
<211>  21
<212>  DNA
<213>  Artificial Sequence

<220>
<223>  Primer

<400>  8
tccaacatca catccaatcc a                                                 21


<210>  9
<211>  143
<212>  DNA
<213>  Homo sapiens

<400>  9
agggagtaga gaaaaagtag gaagatgagt tagaaggcta tcattgggat gtgtaagaga      60

tgaaggagag tttagaccag ggtgataatg agtgtgttgg gaaatagacg aagatagaac     120

agtggattgg atgtgatgtt gga                                              143


<210>  10
<211>  34
<212>  DNA
<213>  Artificial  Sequence

<220>
<223>  Primer

<400>  10
attgggatgt gtaagagatg aaggagagtt taga                                   34


<210>  11
<211>  34
<212>  DNA
<213>  Artificial  Sequence

<220>
<223>  Fluorescent Labeling Primer

<400>  11
attgggatgt gtaagagatg aaggagagtt taga                                   34


<210>  12
<211>  30
<212>  DNA
<213>  Artificial  Sequence

<220>
<223>  Fluorescent Labeling Primer

<400>  12
agggtgataa tgagtgtgtt gggaaataga                                        30